# EUROPEAN PATENT APPLICATION

(11) **EP 3 450 981 A1**
(43) Date of publication of application: **06.03.2019**
(21) Application number: 17188204.6
(22) Date of filing: 28.08.2017
(51) Int. Cl.: G01N 33/569, C12Q 1/68

(54) **BTNL8 AS A MARKER FOR TREGS**

(71) Applicant: Trizell GmbH, 20354 Hamburg (DE)
(72) Inventor: The designation of the inventor has not yet been filed
(74) Representative: Uexküll & Stolberg

(57) **Abstract**

The present invention relates to a method for detecting regulatory T cells (Tregs) in the blood of a subject by use of a novel marker. The method includes the detection of at least one commonly known marker for regulatory T cells (Tregs) in combination with the detection of the marker BTNL8. The method allows the reliable detection and quantification of different Treg populations which are involved in important immunological functions, such as immunosuppression and maintenance of immune tolerance. The invention also provides a method for monitoring the treatment of a subject suffering from an immunological disease or condition which makes use of the novel marker. The invention also relates to the use of a means that allow the specific detection of BTNL8 for detecting Tregs. Kits comprising detection means for detecting commonly known markers of Tregs along with BTNL8 are also provided.

SEQUENCE LISTING

<110>  Trizell GmbH

<120>  BTNL8 as a marker for Tregs

<130>  P 100735

<160>  2

<170>  PatentIn version 3.5

<210>  1
<211>  2239
<212>  DNA
<213>  Homo sapiens

<400>  1
tagacattta tgcagtggtt caaagtctag agtccctaca cttctggtac atgacagctg      60

tgtctcgatg gagtagactc tcagaacagc gcagtttgcc ctccgctcac gcagagcctc     120

tccgtggctt ccgcaccttg agcattaggc cagttctcct cttctctcta atccatccgt     180

cacctctcct gtcatccgtt tccatgccgt gaggtccatt cacagaacac atccatggct     240

ctcatgctca gtttggttct gagtctcctc aagctgggat cagggcagtg gcaggtgttt     300

gggccagaca agcctgtcca ggccttggtg ggggaggacg cagcattctc ctgtttcctg     360

tctcctaaga ccaatgcaga ggccatggaa gtgcggttct tcaggggcca gttctctagc     420

gtggtccacc tctacaggga cgggaaggac cagccattta tgcagatgcc acagtatcaa     480

ggcaggacaa aactggtgaa ggattctatt gcggaggggc gcatctctct gaggctggaa     540

aacattactg tgttggatgc tggcctctat gggtgcagga ttagttccca gtcttactac     600

cagaaggcca tctgggagct acaggtgtca gcactgggct cagttcctct catttccatc     660

acgggatatg ttgatagaga catccagcta ctctgtcagt cctcgggctg gttcccccgg     720

cccacagcga agtggaaagg tccacaagga caggatttgt ccacagactc caggacaaac     780

agagacatgc atggcctgtt tgatgtggag atctctctga ccgtccaaga gaacgccggg     840

agcatatcct gttccatgcg gcatgctcat ctgagccgag aggtggaatc cagggtacag     900

ataggagata cctttttcga gcctatatcg tggcacctgg ctaccaaagt actgggaata     960

ctctgctgtg gcctattttt tggcattgtt ggactgaaga ttttcttctc caaattccag    1020

tgtaagcgag agagagaagc atgggccggt gccttattca tggttccagc agggacagga    1080

tcagagatgc tcccacatcc agctgcttct cttcttctag tcctagcctc caggggccca    1140

ggcccaaaaa aggaaaatcc aggcggaact ggactggaga agaaagcacg gacaggcaga    1200

attgagagac gcccggaaac acgcagtgga ggtgactctg gatccagaga cggctcaccc    1260

gaagctctgc gtttctgatc tgaaaactgt aacccataga aaagctcccc aggaggtgcc    1320

tcactctgag aagagattta caaggaagag tgtggtggct tctcagagtt tccaagcagg    1380

gaaacattac tgggaggtgg acggaggaca caataaaagg tggcgcgtgg gagtgtgccg 1440

ggatgatgtg gacaggagga aggagtacgt gactttgtct cccgatcatg ggtactgggt 1500

cctcagactg aatggagaac atttgtattt cacattaaat ccccgtttta tcagcgtctt 1560

ccccaggacc ccacctacaa aaataggggt cttcctggac tatgagtgtg ggaccatctc 1620

cttcttcaac ataaatgacc agtcccttat ttataccctg acatgtcggt ttgaaggctt 1680

attgaggccc tacattgagt atccgtccta taatgagcaa aatggaactc ccatagtcat 1740

ctgcccagtc acccaggaat cagagaaaga ggcctcttgg caaagggcct ctgcaatccc 1800

agagacaagc aacagtgagt cctcctcaca ggcaaccacg cccttcctcc ccaggggtga 1860

aatgtaggat gaatcacatc ccacattctt ctttagggat attaaggtct ctctcccaga 1920

tccaaagtcc cgcagcagcc ggccaaggtg gcttccagat gaagggggac tggcctgtcc 1980

acatgggagt caggtgtcat ggctgccctg agctgggagg gaagaaggct gacattacat 2040

ttagtttgct ctcactccat ctggctaagt gatcttgaaa taccacctct caggtgaaga 2100

accgtcagga attcccatct cacaggctgt ggtgtagatt aagtagacaa ggaatgtgaa 2160

taatgcttag atcttattga tgacagagtg tatcctaatg gtttgttcat tatattacac 2220

tttcagtaaa aaaaaaaaa 2239

<210> 2
<211> 347
<212> PRT
<213> Homo sapiens

<400> 2

Met Ala Leu Met Leu Ser Leu Val Leu Ser Leu Leu Lys Leu Gly Ser
1               5                   10                  15

Gly Gln Trp Gln Val Phe Gly Pro Asp Lys Pro Val Gln Ala Leu Val
            20                  25                  30

Gly Glu Asp Ala Ala Phe Ser Cys Phe Leu Ser Pro Lys Thr Asn Ala
        35                  40                  45

Glu Ala Met Glu Val Arg Phe Phe Arg Gly Gln Phe Ser Ser Val Val
    50                  55                  60

His Leu Tyr Arg Asp Gly Lys Asp Gln Pro Phe Met Gln Met Pro Gln
65                  70                  75                  80

Tyr Gln Gly Arg Thr Lys Leu Val Lys Asp Ser Ile Ala Glu Gly Arg
                85                  90                  95

Ile Ser Leu Arg Leu Glu Asn Ile Thr Val Leu Asp Ala Gly Leu Tyr
            100                 105                 110

Gly Cys Arg Ile Ser Ser Gln Ser Tyr Tyr Gln Lys Ala Ile Trp Glu
        115                 120                 125

Leu Gln Val Ser Ala Leu Gly Ser Val Pro Leu Ile Ser Ile Thr Gly
    130                 135                 140

Tyr Val Asp Arg Asp Ile Gln Leu Leu Cys Gln Ser Ser Gly Trp Phe
145                 150                 155                 160

Pro Arg Pro Thr Ala Lys Trp Lys Gly Pro Gln Gly Gln Asp Leu Ser
                165                 170                 175

Thr Asp Ser Arg Thr Asn Arg Asp Met His Gly Leu Phe Asp Val Glu
            180                 185                 190

Ile Ser Leu Thr Val Gln Glu Asn Ala Gly Ser Ile Ser Cys Ser Met
        195                 200                 205

Arg His Ala His Leu Ser Arg Glu Val Glu Ser Arg Val Gln Ile Gly
    210                 215                 220

Asp Thr Phe Phe Glu Pro Ile Ser Trp His Leu Ala Thr Lys Val Leu
225                 230                 235                 240

Gly Ile Leu Cys Cys Gly Leu Phe Phe Gly Ile Val Gly Leu Lys Ile
                245                 250                 255

Phe Phe Ser Lys Phe Gln Cys Lys Arg Glu Arg Glu Ala Trp Ala Gly
            260                 265                 270

Ala Leu Phe Met Val Pro Ala Gly Thr Gly Ser Glu Met Leu Pro His
        275                 280                 285

Pro Ala Ala Ser Leu Leu Leu Val Leu Ala Ser Arg Gly Pro Gly Pro
    290                 295                 300

Lys Lys Glu Asn Pro Gly Gly Thr Gly Leu Glu Lys Lys Ala Arg Thr
305                 310                 315                 320

Gly Arg Ile Glu Arg Arg Pro Glu Thr Arg Ser Gly Gly Asp Ser Gly
                325                 330                 335

Ser Arg Asp Gly Ser Pro Glu Ala Leu Arg Phe
            340                 345

## Description

The present invention relates to a method for detecting regulatory T cells (Tregs) in the blood of a subject by use of a novel Treg marker. The method includes the detection of at least one commonly known marker for regulatory T cells in combination with the detection of the cell-surface marker BTNL8. The method allows the reliable detection and quantification of Treg populations which are involved in important immunological functions, such as immunosuppression and maintenance of immune tolerance. The invention also provides a method for monitoring the treatment of a subject suffering from an immunological disease or condition which makes use of the novel marker. The invention also relates to the use of a means that allows the specific detection of BTNL8 for detecting Tregs. Kits comprising means for detecting commonly known markers of Tregs along with BTNL8 are also provided.

### BACKGROUND OF THE INVENTION

The transfer of immunoregulatory cells from an immunologically tolerant donor to a non-tolerant individual as a means of establishing tolerance is presently receiving serious attention [1]. Several classes of immunoregulatory cells [2] are currently being developed as adjunct immunosuppressive agents for use in solid organ transplantation, including different sorts of regulatory T cells (Tregs) and suppressive myeloid cells [3-5].

Tregs are one type of regulatory cells that have been proven particularly useful in this regard owing to their immunosuppressive effects. Tregs are a subgroup of T cells which have the capability of suppressing immune responses and promoting immunologic tolerance to cells or organs from allogeneic donors. In recent years, the roles of Tregs in controlling a variety of physiological and pathological immune responses have been demonstrated, and the use of Tregs for the treatment of autoimmune diseases, solid organ transplantation, and hematopoietic stem cell transplantation has been suggested. Several studies are currently underway which isolate Tregs from donors, expand these cells *ex vivo* and infuse them into the patient to be treated where they suppress immunological rejection of a transplant. In such approaches, monitoring of the suppressive Tregs infused into the patient is important for predicting the treatment efficiency. Accordingly, there is a need for markers that allow the detection and monitoring of Tregs after their administration into a patient.

Such markers would not only be useful for treatments that rely on the administration of isolated Tregs, but also for therapeutic approaches which are based on other types of immunoregulatory cells which have the capability of inducing Tregs in the patient. For example, human regulatory macrophages (Mregs) are a cell type that is already far advanced in its development as a cell-based medicinal product [6]. As early as 2008, living-donor kidney transplant recipients were treated pre-operatively with crude Mreg preparations with the intention of reducing their long-term dependence on immunosuppressive drug treatment [7]. Mregs reflect a unique state of macrophage differentiation, distinguished from macrophages in other activation states by their mode of derivation, relatively stable phenotype and potent suppressor function [8-9]. Human Mregs develop from M-CSF-stimulated CD14+ peripheral blood monocytes cultured with high concentrations of human serum [10] through a time-dependent process that requires contact with a plastic surface, ligation of CD16 by serum immunoglobulin and exposure to other serum factors [11]. Under these culture conditions, human Mregs gradually acquire their characteristic spreading morphology and cell-surface phenotype. Human Mregs prevent mitogen-stimulated allogeneic T cell proliferation in vitro through IFN-γ-induced indoleamine 2,3-dioxygenase (IDO) activity, as well as mediating a contact-dependent deletion of activated allogeneic T cells [12]. The simplicity of Mreg production and their stable suppressive activity render them suitable as a cell-based therapeutic product for use in immunosuppressive therapy. Preclinical experiments in a heterotopic mouse heart transplant model demonstrated the capacity of donor-strain Mregs administered by i.v. injection to prolong allograft survival in immunocompetent, fully-mismatched recipients [13]. Following i.v. injection into mice, allogeneic Mregs rapidly accumulated in lung, liver and spleen, but not lymph nodes, where they persisted for up to 4 weeks. Mregs exert a graft-protective effect that endures beyond their lifespan, and this effect is mediated by Tregs that are induced in the recipient. Thus, as immunosuppressive Tregs appear to play a crucial role also in Mreg-induced immunologic responses, their specific detection and quantification is of importance for assessing the effectiveness of an Mreg therapy.

Marker patterns which are characteristic for immunosuppressive Tregs have been described in the art and include CD3⁺, CD4⁺, CD25⁺, CD127^{low}, LAP⁺, GARP⁺ and FOXP3⁺. However, the detection of Tregs by the above markers is often problematic and leads to unclear results. For example, the markers LAP and GARP are expressed on a low level which means that the distinguishing weakly positive and negative cells is sometimes challenging. As a consequence, it has been found that a considerable part of immunosuppressive Tregs escape cell sorting methods such as fluorescence-activated cell sorting (FACS) or magnetic bead-based separation.

Another drawback of the above marker pattern is that the marker FOXP3 is an intracellular transcription factor which means that the T cell has to be killed to allow an antibody to permeate the cell and enter the nucleus where said marker is expressed [14]. Accordingly, FOXP3 does not allow viable Treg cells to be sorted by FACS or magnetic bead-based sorting. In addition, effector T cells up-regulate FOXP3 expression upon their activation [15]. Similarly, the markers CD3⁺, CD4⁺, CD25⁺ and CD127^{low} are not specific for regulatory Tregs, but are common to other types of human T cells.

Owing to the fundamental relevance of Tregs for the treatment of a number of diseases and disorders, there is a need to further identify specific markers that are useful for detecting and monitoring immunosuppressive Tregs. It has now been found in the course of the present invention that immunosuppressive Tregs appear to express higher levels of butyrophilin-like protein 8 (BTNL8) at their surface or within intracellular reserves than effector T cells or other types of T cells. Therefore, BTNL8 can be used for the specific identification, detection, isolation, quantification, monitoring and/or enrichment of immunosuppressive Tregs.

### DESCRIPTION OF THE INVENTION

The present invention is based on the insight that BTNL8 is present on the surface of immunosuppressive Tregs. Therefore, in a first aspect, the invention provides a method for detecting immunosuppressive Tregs in a subject, said method comprising detecting in a cell sample that has been obtained from the subject:
(a) at least one marker which is known to be associated with Tregs;
(b) the marker BTNL8.

The method of the invention relies on the detection of the novel marker BTNL8 in combination with at least one commonly known marker that is known to be expressed by activated immunosuppressive Tregs and is to some extent specific for these cells.

BTNL8 is a little-studied cell surface receptor belonging to the 4-member butyrophilin-like gene family in humans, which is closely related to the butyrophilin family, both of which families are structurally and functionally related to the B7-costimulatory molecules. BTNL8 is a type-I membrane protein with an extracellular region comprising an IgV and IgC domain [16]. It is a cell-surface receptor that serves as a costimulatory or coinhibitory ligand for a so far uncharacterized receptor expressed by T cells. BTNL8 is expressed in the form of two different variants: a BTN-like variant of 57 kDa and a B7-like variant of about 37 kDa. The BTN-like variant of 57 kDa incorporates an intracellular B30.2 domain that is lacking from the 37-kD isoform. Expression of both BTNL8 mRNA splice variants was previously reported in neutrophils and eosinophils [17], as well as enterocytes, but BTNL8 expression in T cells, B cells, NK cells or monocytes has not previously been reported. In the course of the experiments reported in the below examples, it was found that the B7-like variant of 37 kDa is highly up-regulated by induced Tregs (iTregs) that have been activated by co-culturing them with Mreg cells. Accordingly, if it is referred to BTNL8 in the present application, the B7-like variant of BTNL8 having a molecular weight of about 37 kDa is meant. The nucleotide sequence of the B7-like splicing variant of BTNL8 is set forth in SEQ ID NO:1 herein. The amino acid sequence of the BTNL8 is set forth in SEQ ID NO:2.

To achieve specificity for Tregs, the detection of the marker BTNL8 is combined with the simultaneous or subsequent detection of at least one marker which is known to be expressed by immunosuppressive Tregs. Markers characteristic for immunosuppressive Tregs are known in the art and comprise, but are not limited to, GARP, LAP, CD25, CD127, CD121a, CD121b, and FoxP3. By combining one or more of these markers with the novel BTNL8 marker, a highly specific detection of immunosuppressive Tregs in a sample is feasible. Unlike FoxP3, the novel marker BTNL8 is expressed on the surface of immunosuppressive Tregs, and it may therefore be used in many settings as a surrogate marker for FoxP3.

In one embodiment of the method of the invention, BTNL8 is used in combination with CD25. As used herein, CD25 refers to the interleukin-2 (IL-2) receptor alpha chain, a protein which is included in humans by the IL2RA gene [18]. The IL2 receptor alpha (IL2RA) chain, together with the IL2 receptor beta (IL2RB) chain and the IL2 receptor gamma chain (IL2RG) form an IL-2 receptor of high affinity. CD25 is a type I receptor protein which is present on T cells, B cells, some thymocytes, myeloid precursors, and oligodendrocytes. In such embodiment, immunosuppressive Tregs can be identified by the marker profile BTNL8⁺ CD25⁺.

In another embodiment of the method of the invention, BTNL8 is used in combination with CD127. In the context with the present invention, CD127 refers to the alpha subunit of the interleukin-7 receptor [19-20]. The heterodimeric interleukin-7 receptor consists of two distinct protein chains, namely interleukin-7 receptor-α (CD127) and common-y chain receptor (CD132). While CD127 is unique for the interleukin-7 receptor, the common-y chain receptors are shared with various cytokines, including interleukin-2, -4, -9, and -15. CD127 is expressed on various cell types, including naive and memory T cells. Importantly, Tregs are characterized by CD127 expression which is significantly lower compared to that observed in conventional T cells. In such embodiment, immunosuppressive Tregs can be identified by the marker profile BTNL8⁺ CD127^{low}.

In yet another embodiment of the method of the invention, BTNL8 is used in combination with the marker glycoprotein A repetitions predominant (GARP). This 80-kDa transmembrane protein consists of 662 amino acids and has an extracellular region composed primarily of 20 leucine-rich repeats [21-22]. GARP is supposed to play an important role in cell development, but its actual function is still not known. It has been shown that GARP is expressed on the surface of immunosuppressive Tregs. In such embodiment, immunosuppressive Tregs can be identified by the marker profile BTNL8⁺ GARP⁺.

In yet another embodiment of the method of the invention, BTNL8 is used in combination with the latency-associated peptide (LAP) as a marker. LAP is a protein that is normally associated with TGF-β to keep TGF-β inactive. TGF-β is secreted by many cell types in a latent form in which it is complexed with two polypeptides [23-24], namely latent TGF-beta binding protein (LTBP) and latency-associated peptide (LAP). After its secretion, serum proteinases catalyze the release of active TGF-β from the complex. TGF-β also plays a crucial role both in T-cell regulation and differentiation. In such embodiment, immunosuppressive Tregs can be identified by the marker profile BTNL8⁺ LAP⁺.

In yet another embodiment of the method of the invention, BTNL8 is used in combination with CD121a. As used herein, CD121a refers to the interleukin 1 receptor type I, a single-pass, type I transmembrane glycoprotein that belongs to the interleukin 1 receptor (IL1R) family. CD121a has been found to be responsible for triggering IL-1-mediated inflammation [25]. In such embodiment, immunosuppressive Tregs can be identified by the marker profile BTNL8⁺ CD121a⁺.

In yet another embodiment of the method of the invention, BTNL8 is used in combination with CD121b. As used herein, CD121b refers to the interleukin 1 receptor type II which likewise belongs to the interleukin 1 receptor (IL1R) family. CD121b is a decoy receptor for interleukin-1α, interleukin-1β, and interleukin 1 receptor antagonist, thereby preventing them from binding to their regular receptors and inhibiting the transduction of their signaling. In such embodiment, immunosuppressive Tregs can be identified by the marker profile BTNL8⁺ CD121b⁺.

Typically, the detection of markers will also include markers which are commonly known to be present on T cells or T cell subpopulations, such as T helper cells. Therefore, it is particularly preferred that the detection of Tregs also comprises the T cell markers CD3 and CD4. The markers CD3 and CD4 are well known markers that are widely used for the detection and sorting of T cells. While CD3 is present on the surface of all T cells, CD4 is expressed by T helper cells (to which Tregs belong). Accordingly, the positive detection of CD3 and CD4 allows the conclusion that the cell having both surface markers is a T helper cell.

The inventors found that Mregs strongly induced TIGIT expression in co-cultured T cells through an IDO-dependent mechanism. Accordingly, in another embodiment, the method of the invention comprises, apart from the detection of the at least one known Treg marker and BTNL8, also the detection of TIGIT. In still another embodiment, the method of the invention comprises, apart from the detection of the at least one known Treg marker and BTNL8, also the detection of the marker HELIOS.

Based on the present disclosure, the skilled person will be readily able to design different marker patterns which reliably identify and detect Tregs. While it is in principle sufficient to combine the new marker BTNL8 with one additional Treg marker, it is preferred that more than two markers are simultaneously used in order to improve specificity of the detection. In particular, it will be preferred to use CD4 as an additional marker to ensure that the detected cell belongs to the group of T helper cells. Preferred marker patterns for use in the practice of the present invention comprise the detection of the following markers:
(a) CD4, GARP, and BTNL8,
(b) CD4, LAP, and BTNL8,
(c) CD4, GARP, LAP, and BTNL8,
(d) CD4, CD25, GARP, and BTNL8,
(e) CD4, CD25, LAP, and BTNL8,
(f) CD4, CD25, GARP, LAP, and BTNL8,
(g) CD4, CD127, GARP, and BTNL8,
(h) CD4, CD127, LAP, and BTNL8,
(i) CD4, CD127, GARP, LAP, and BTNL8,
(j) CD4, CD121a, GARP, and BTNL8,
(k) CD4, CD121a, LAP, and BTNL8,
(l) CD4, CD121a, GARP, LAP, and BTNL8,
(m) CD4, CD121b, GARP, and BTNL8,
(n) CD4, CD121b, LAP, and BTNL8,
(o) CD4, CD121b, GARP, LAP, and BTNL8.

According to the invention, the detection of Tregs can be performed on a qualitative or quantitative basis. For some applications it will be sufficient to detect the presence of certain cells having the desired marker pattern. However, for most applications it will be preferred that the target cells are quantitatively assessed. Accordingly, the above method may comprise a further step in which the Tregs are quantified. Quantification of the cells can be achieved by different means which mainly depend on the specific detection method. For example, the quantity of detection by RT-PCR or flow cytometry is well known in the art.

The cell sample used in the method of the present invention may be any sample that contains or is supposed to contain immunosuppressive Tregs. For example, the cell sample can be a tissue sample which has been obtained from the subject to be tested, e.g., by core needle biopsy, fine needle aspiration, and the like. Before being used in the methods of the invention, the sample can be processed to render the sample eligible for marker analysis, e.g., by cell lysis and subsequent RNA purification, by dilution with a suitable buffer, or by staining the cells, e.g. with fluorescence-labeled antibodies. In a particularly preferred embodiment, the cell sample obtained from the subject is a blood sample or a sample which has been derived from a blood sample, such as a serum or plasma sample.

In a particularly preferred embodiment of the invention, the method aims at the detection of Tregs which have been induced by the administration of Mregs to the subject in need thereof. In that case the sample will be derived from a subject to whom a cell-based product was administered before detecting said Tregs, and the cell-based product preferably comprised Mregs.

In a particularly preferred embodiment, detection of the markers in steps (a) and (b) involves measuring BTNL8 and/or at least one of the above discussed markers which are known to be associated with Tregs at the transcriptional level. Suitable methods for monitoring expression of the BTNL8 gene at the transcriptional level preferably include those which allow for the quantitative or semi-quantitative detection of mRNA levels, for example, quantitative RT-PCR (e.g., TaqMan™ RT-PCR), real-time RT-PCR, Northern-Blot analysis or other methods which are generally described, e.g., in Sambrook et al. (eds.) Molecular Cloning: A Laboratory Manual, 2nd ed., Cold Spring Harbor Laboratory Press, 1989.

The detection of expression levels at the transcriptional level usually requires as a first step the isolation of mRNA from the cell sample of the subject, e.g. from a blood sample. Methods for isolating RNA, such as mRNA, are well known in the art and are discussed in detail in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Such methods regularly involve the lysis of the cells or tissues obtained from the subject to be tested. Cell lysis may be performed by use of detergents which are capable of disrupting the plasma membrane of the cells. For example, buffers containing guanidine thiocyanate and/or SDS may be used for cell lysis. The methods may comprise steps in which the DNA of the cells is enzymatically digested in order to obtain pure RNA without traces of DNA which might interfere with further downstream applications, such as the monitoring of expression levels. Inhibitors of enzymes which lead to the degradation of the RNA may also be added to the lysis buffer. Kits for preparing highly purified RNA are available from several manufacturers, such as Qiagen, Ambion, Stratagene, Clontech, Invitrogen, Promega, and others.

The RNA isolated from the cell or tissue sample by use of commercial kits will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex™ matrix can be performed, as mRNAs contain a poly(A) tail at their 3' terminus (see, for example, Ausubel et al. (1994), Current Protocols in Molecular Biology, Current Protocols Publishing, New York). Poly(A)+ mRNA which has bound to the affinity matrix can be eluted using 2 mM EDTA/0.1% SDS.

One commonly used method for detecting expression at the transcriptional level is RT-PCR. In this method, an mRNA template is transferred into cDNA by a reverse transcription (RT) reaction. The reverse transcription of the RNA template is catalyzed by a reverse transcriptase, and the reaction can be primed by specific oligonucleotide primers, or, alternatively, by oligo-dT primers.

The cDNA is then used as template for a subsequent PCR reaction. In the subsequent PCR step, the cDNA is amplified by use of specific oligonucleotide primers and a polymerase enzyme, e.g., a Taq polymerase. In a preferred embodiment, the RT-PCR reaction is performed as a real-time RT-PCR, which enables the detection and the simultaneous quantification of amplified DNA in real-time. Quantification occurs either as an absolute number of copies or as a relative amount normalized by use of additional gene expression products.

In a further preferred embodiment, a TaqMan RT-PCR is used for determining the expression levels of the BTNL8 gene and/or the other Treg marker genes. The TaqMan RT-PCR is a fluorophore-based RT-PCR method which detects accumulation of a specific, amplified product during PCR. In TaqMan RT-PCR, RNA is first transferred into cDNA by a reverse transcriptase. In the subsequent PCR reaction, a single-stranded oligonucleotide probe is added which is complementary to a segment of 10-60 nucleotides within the DNA template and located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR, the 5' exonuclease activity of the Taq polymerase cleaves the oligonucleotide probe which is hybridized to the template DNA, thereby spatially separating the fluorophore and the quencher dye. Fluorescence is detected during PCR cycling; it is directly proportional to the fluorophore released and the amount of DNA template present in the PCR. Every fluorophore-quencher pair known in the art can be used in the methods of the present invention. Examples of suitable fluorophores are FAM (6-carboxyfluorescin), TET (tetrachlorofluorescin) or VIC. A suitable quencher dye is TAMRA (tetramethylrhodamine). The construction and labeling of oligonucleotides to be used as probes in a TaqMan approach are described in great detail in the literature. A TaqMan approach RT-PCR reaction can be carried out using, e.g., the ABI PRISM 7700 system (Perkin-Elmer/Applied Biosystems, Foster City, Calif., USA), or the Lightcycler system (Roche Molecular Biochemicals, Manheim, Germany).

A microarray is another commonly used tool in expression profiling. A microarray refers to the orderly arrangement of spatially resolved probes, for example nucleic acid probes, on a substrate. Such arrays can contain at least one, and preferably more than one, oligonucleotide which is complementary to the gene of interest, e.g. the BTNL8 gene and/or the other Treg marker genes, and thus hybridizes to the respective gene sequence or its transcript. The substrate is preferably a solid substrate having a surface with a plurality of probes attached in different known locations (spots). The spots on a microarray are normally either printed on the microarrays or synthesized by photolithography or by ink-jet printing. On a common microarray, there may be several thousand spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10,000, at least 10⁵, at least 10⁶, at least 10⁷ oligonucleotides or fragments per cm². The support can be a glass or plastic slide or membrane on the surface of which the probes are attached at fixed locations or spots.

The primers or probes used in the PCR or RT-PCR reactions mentioned herein will be designed to allow the specific hybridization to and the subsequent amplification of the target sequence within the respective Treg marker gene, the BTNL8 gene. Based on the present disclosure, the skilled person will be readily able to design oligonucleotide primers and/or probes which can be used for detecting the expression of the respective Treg marker gene. Methods for designing sequence-specific oligonucleotide primers for PCR or RT-PCR are discussed in great detail in the scientific literature, e.g. in Dieffenbach and Dveksler, "PCR Primer", A Laboratory Manual, Cold Spring Harbor Laboratory Press, New York, 2003. The parameters to be considered when designing PCR primers include, for example, the number of nucleotides, the G/C content of the primer, the melting temperature, the presence of complementary nucleotides which may lead to secondary structures, and the like. The oligonucleotides for use in the methods of the present invention preferably have a length of at least 8 nucleotides, more preferably at least 10, 12, 14, 16, 18, 20, 22, 24, 26, 28, 30, 32, 34, 36, 38, 40, 42, 44, 46, 48 or 50 nucleotides. The oligonucleotide primers can be prepared by any suitable technique known in the art. For example, they may be derived synthetically, for example by the phosphoamidite method. Apart from oligomers or polymers which contain the naturally occurring nucleotide bases adenine, thymine (uridine), cytosine or guanine, oligonucleotide primers of the invention may also include modified bases, such as 5-methylcytosine, 5-hydroxymethylcytosine, and the like.

Probes and primers for the detection of BTNL8 can be designed according to routine methods based on the BTNL8 sequence set forth in SEQ ID NO:1, which is the coding sequence of the human BTNL8 gene, i.e. the sequence obtained after splicing of the mRNA. Preferably, the methods of the invention which are directed to the detection of expression levels make use of the mRNA sequence corresponding to the human DNA sequence depicted in SEQ ID NO:1. It will be appreciated that the polynucleotide sequence of the human BTNL8 gene may exhibit a limited number of sequence deviations resulting, e.g., from naturally occurring polymorphisms. Encompassed by the term BTNL8 gene are thus also polynucleotides which result in a mRNA molecule exhibiting at least about 90%, 95%, 96%, 97%, 98%, 99% or more sequence identity to the sequence of SEQ ID NO:1. Computer programs for determining the degree of identity between nucleotide sequences are available, e.g., in the Wisconsin Sequence Analysis Package, Version 8 (available from Genetics Computer Group, Madison, USA) and include, e.g., the programs BESTFIT, FASTA and GAP, which are based on the algorithm of Smith and Waterman. These programs can be used with the standard parameters, as recommended by the manufacturer.

Where high levels of BTNL8 mRNA in a T cell are detected, it can be assumed that this cell is a Treg cell. While it is possible that also non-Treg cells, such as effector T cells, express BTNL8 to some degree, such expression will be comparatively low. According to the invention, it is hence preferred that the expression of BTNL8 (or any other Treg marker) by non-Treg cells, in particular by an effector T cell, is used as a negative control. Measuring a BTNL8 expression at an RNA level which is at least about 50%, at least about 75%, at least about 150%, at least about 200%, at least about 250%, at least about 300%, at least about 350%, at least about 400%, at least about 450%, at least about 500%, at least about 550%, at least about 600%, at least about 750%, or at least about 1000% higher than the respective amounts in the negative control, i.e. in the non-Treg cells, such as effector T cells, clearly indicates that the tested cell is an immunosuppressive Treg cell.

In yet another particularly preferred embodiment, detection of the Treg markers in steps (a) and (b) involves the detection of Treg marker gene expression at the translational level. This means that the protein level of the respective Treg marker, such as BTNL8, is determined. Treg protein levels may be determined by any suitable method which is able to specifically detect the BTNL8 protein in a biological sample. The detection of the protein may be based on a molecule that binds specifically to the protein or it may be based on the separation of the protein from other proteins that are present in the sample. Molecules that specifically bind to Treg marker proteins include antibodies and antibody fragments with binding activity for the Treg markers such BTNL8. Numerous antibodies have been prepared and can be commercially chased which are directed to Treg marker proteins like GARP, LAP, CD25, CD127, CD121a, CD121b, and FoxP3. Such antibodies or fragments thereof may be used for detection of the Treg marker protein in a Western blot, quantitative Western blot, enzyme-linked immunosorbent-assay (ELISA), polarization analysis, (quantitative) surface plasmon resonance (SPR), or in immunohistochemical methods, including quantitative electronmicroscopic methods. In a particularly preferred embodiment of the invention, the detection in steps (a) and (b) of the above methods uses an ELISA. Other methods which are able to detect specific binding include, for example, fluorescence resonance energy transfer (FRET). Methods which allow the quantitative detection of the Treg marker protein by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

As stated above, it is possible that also effector T cells may produce BTNL8 in low amounts. Accordingly, protein levels of non-Treg cells, such as effector T cells, should also be measured to provide a negative control. If the protein levels for tested cells reveal that the BTNL8 expression is higher than the negative control, this allows the conclusion that the tested cell is a Treg. Specifically, measuring a BTNL8 expression at the protein level which is at least about 50%, at least about 75%, at least about 150%, at least about 200%, at least about 250%, at least about 300%, at least about 350%, at least about 400%, at least about 450%, at least about 500%, at least about 550%, at least about 600%, at least about 750%, or at least about 1000% higher than the respective amounts in the negative control, i.e. in the non-Treg cells, such as effector T cells, clearly indicates that the tested cell is an immunosuppressive Treg cell.

In an even more preferred embodiment of the invention, detection of the Treg markers in steps (a) and (b) of the method of the invention involves flow cytometry. Flow cytometry is a widely used method for analyzing the expression of cell surface markers and intracellular molecules. It is routinely used for applications like cell counting, cell sorting, and biomarker profiling. In particular it can be used for defining different cell types in a heterogeneous cell population. Flow cytometry is predominantly used to measure fluorescence intensity produced by fluorescent-labeled antibodies that detect markers on the cell surface. Although it can also be used for the detection of intracellular markers, such detection is less desirable, since the antibody must permeate the cell which normally kills the cell. This prevents the detection of intracellular markers for cell sorting applications which aims at the provision of viable homogeneous cell populations.

The insight that BTNL8 is expressed on the surface of Tregs provides for the opportunity of monitoring the treatment of an immunological disease which aims at the induction of Tregs. Accordingly, in a second aspect of the present invention, a method for monitoring or evaluating the treatment of an immunological disease or condition in a subject is provided, said method comprising
(a) providing a sample that has been obtained from said subject after treatment of said immunological disease or condition has been initiated;
(b) carrying out the detection method described above; and
(c) quantifying the BTNL8-expressing Tregs;
wherein an increase in the number of BTNL8-expressing Tregs after initiation of treatment indicates that the treatment is effective.

This method allows determining whether the treatment of an immunological disease or disorder that seeks to increase the number of Tregs in a patient is effective, as it specifically allows the detection and quantification of such Tregs. In the first step of said method, a sample is provided from a subject who suffers from an immunological disease or condition. Preferably, this sample is a blood or blood-derived sample such as a plasma or serum sample. The treatment of the immunological disease or condition in the subject has already begun. For example, the subject may have received medication for the treatment of the immunological disease or condition 6 hours, 12 hours, 24 hours, or 48 hours prior to the collection of the sample. The sample can be obtained from the subject by conventional methods as discussed elsewhere herein. In a subsequent step, the detection method according to the first aspect of the invention is performed to detect BTNL8-expressing Tregs. The BTNL8-expressing Tregs are then quantified and optionally compared to a negative control. A suitable negative control could be a sample from the same subject prior to start of the treatment, i.e. the subject who has not received any medication. An increase in the number of BTNL8-expressing Tregs indicates that the administered medication, for example an Mreg-based composition, is effective and induces Tregs.

The treatment is effective in particular if the number of BTNL8-expressing Tregs quantified by the above method is at least 2-fold, at least 5-fold, at least 10-fold, at least 20-fold, at least 50-fold, at least 75-fold, at least 100-fold, at least 150-fold, at least 250-fold, at least 500-fold, or at least 1000-fold higher compared to the number of BTNL8-expressing Tregs in the untreated subject, i.e. the subject before the treatment had been started.

The treatment of the immunological disease or disorder can comprise the administration of Tregs or, alternatively, the administration of compounds or cells that are capable of inducing Tregs. The treatment preferably comprises the administration of a cell-based composition. The cell-based composition may comprise, for example, suppressive myeloid cells or Mregs. Immunological diseases or disorders that might be treated or ameliorated by the induction or administration of Tregs include treatments which seek to suppress transplant rejection or prolong transplant survival in a subject receiving a transplant, such as an organ, tissue or cell transplant. The organ to be transplanted may be, for example, a kidney, liver, heart, lung, or pancreas. It is particularly preferred that the organ to be transplanted to the recipient is a human organ. The transplant may also be a tissue transplant. The tissue to be transplanted will preferably be a human tissue, such as an intestinal, cornea, skin, composite tissue, bone marrow, or pancreatic islet tissue. Where the transplant is a cell transplant, the nature of the cell to be transplanted is generally not limited, but it is preferred that the cell to be transplanted is selected from the group consisting of an adult stem cell transplant, an isolated hepatocyte transplant, a haematopoietic stem cell (HSC) transplant or a leukocyte cell transplant. The transplant will normally be an allogeneic transplant, i.e. a transplant which originates from a donor that, although genetically different, belongs to the same species as the recipient.

The suppression of transplant rejection in the recipient and the induction of acceptance of an allogeneic organ, tissue or cell transplant can be induced by the administration of Mregs. The cells can be administered intravenously by injection or infusion. The injection or infusion can be given either pre-operatively or post-operatively. If the cells are administered pre-operatively, they will be administered to the recipient at least once, preferably twice, and more preferably three times before the operation.

Where Mregs are used for the induction of Tregs, it is preferred that the Mregs are administered to the recipient not earlier than one week before operation, e.g. 6 days, 5 days, 4 days, 3 days, 2 days, or 1 day before operation. If the Mreg cells are administered post-operatively, a first administration will be given preferably within 24 hours after operation, more preferably within 36 hours, 48 hours, 60 hours, or 72 hours after operation. Alternatively, in stably immunosuppressed transplant recipients, Mreg therapy may be administered at any time after transplantation. Alternatively, Mregs may be administered to transplant recipients undergoing acute or chronic transplant rejection. The Mregs are then capable of repelling the T-cell response of the recipient's immune system against the transplant and to persist in the recipient's body (especially spleen, liver, lungs and bone marrow) for a sufficiently long period of time to confer long-term transplant acceptance to the recipient.

Immunological diseases that might be treated by Tregs further include diseases or disorders that are characterized by a deregulated immune status or an excessive inflammatory reaction, in particular chronic inflammatory diseases. Such diseases or disorders include, for example, autoimmune diseases, inflammatory diseases and hypersensitivity reactions. The disease may be a local or systemic autoimmune condition. The type of autoimmune disease to be treated includes systemic lupus erythematosus (SLE), scleroderma, Sjögren's syndrome, polymyositis, dermatomyositis, and other systemic autoimmune conditions; rheumatoid arthritis (RA), juvenile rheumatoid arthritis, and other inflammatory arthritides; ulcerative colitis, Crohn's disease, and other inflammatory bowel diseases; autoimmune hepatitis, primary biliary cirrhosis, and other autoimmune liver diseases; cutaneous small-vessel vasculitis, granulomatosis with polyangiitis, eosinophilic granulomatosis with polyangiitis, Behçet's disease, thromboangiitis obliterans, Kawasaki disease, and other large-, medium- or small-vessel vasculitides of autoimmune aetiology; Multiple sclerosis (MS) and neuroimmunological disorders; Type I diabetes, autoimmune thyroid dysfunction, autoimmune pituitary dysfunction, and other autoimmune endocrinological disorders; haemolytic anaemia, thrombocytopaenic purpura and other autoimmune disorders of the blood and bone marrow; psoriasis, pemphigus vulgaris, pemphigoid and other autoimmune conditions that pertain to the skin.

The treatment may also be directed to acute or chronic inflammatory diseases or diseases with a pathophysiologically significant inflammatory component. The inflammatory disease to be treated may be local or systemic. The type of inflammatory diseases or condition that benefit from Tregs is not limited and includes, but is not limited to arterial occlusive disease, such as peripheral artery occlusive disease (pAOD), critical limb ischemia, arteriosclerosis, cerebral infarction, myocardial infarction, renal infarction, intestinal infarction, angina pectoris, and other conditions caused by arterial occlusion or constriction; microvascular angina, also known as cardiac syndrome X; inflammation-associated systemic metabolic disorders, including Type II diabetes and obesity-related metabolic syndrome; or dermatological diseases, including eczema. Preferably, the inflammatory disease to be treated is characterized by chronic inflammation of the intima of an arterial wall, e.g. myocardial infarction, stroke, critical limb ischemia vasculitis and pAOD. The treatment may also be directed to a hypersensitivity reaction which is preferably selected from the group of asthma, eczema, allergic rhinitis, angioedema, drug hypersensitivity, and mastocytosis.

In a third aspect, the invention relates to method for enriching, isolating or sorting regulatory T cells (Tregs), said method comprising:
(a) providing a sample that contains or is supposed to contain Tregs;
(b) separating Tregs from other cells that are contained in the sample via their expression of BTNL8 and at least one marker which is known to be associated with Tregs, wherein said marker preferably is selected from the group of CD25, CD127, CD121a, CD121b, GARP, LAP and FoxP3.

Preferably, preparation step (b) is performed by use of a compound that has affinity for BTNL8, more preferably an antibody or antibody fragment as described below. In one embodiment, the method can be cell sorting method based on flow cytometry, such as fluorescence-activated cell sorting (FACS). In this method, the Tregs are contacted with a fluorescence-labeled antibody or antibody fragment which is directed to BTNL8 and at least one additional antibody or antibody fragment directed to another Treg marker, preferably from the group of CD25, CD127, CD121a, CD121b, GARP, LAP and FoxP3. BTNL8-expressing Tregs are conducted in a fluid stream through an electronic detection device. The fluid stream is broken into droplets having a size to ensure that each droplet contains no more than one cell. Immediately before breaking the stream breaks into droplets, the fluid stream is conducted through a fluorescence measuring device where the fluorescent signals of the cells are measured. Based on the detected signals, the cells are led in different containers so as to achieve a separation of the differently labeled cells. In another preferred embodiment, the method can involve magnetic bead cell separation. This method uses superparamagnetic nanoparticles having a size of about100 nm for tagging cells. The cells are then captured in columns having affinity for the nanoparticles.

In a fourth aspect, the invention relates to the use of a compound having affinity for the BTNL8 protein for detecting, enriching, isolating and/or quantifying Tregs. The affinity compounds are broadly understood as compounds which bind to BTNL8 on the surface of the cells. The affinity compounds are preferably protein molecules, such as receptors, ligands or antibodies. In a particular preferred embodiment, said affinity compound is an antibody or antibody fragment which is directed to BTNL8. The insight that BTNL8 is expressed on the surface of Tregs provides for the possibility to detect, enrich, isolate and quantify BTNL8-expressing Tregs in an inhomogeneous composition such as a blood sample. Antibodies directed to BTNL8 have been described in the art and are also commercially available from different manufacturers. In the context of the present invention, the term "antibody" has to be understood broadly to include monoclonal antibodies, polyclonal antibodies, multispecific antibodies, such as bispecific antibodies, anti-idiotypic antibodies, chimeric antibodies, and humanized antibodies, as long as they exhibit the desired immunological activity, i.e. specific binding to BTNL8. In particular, the term is to be understood as comprising any kind of artificial antibody molecule that was prepared by grafting techniques and the like. The antibodies for use in the present method may be of any isotype class, such as IgG, IgM, IgA, IgE, and IgD as well as their subclasses, e.g., IgG1, IgG2 and the like.

Apart from whole antibodies, BTNL8-binding fragments of an antibody may also be used. As used herein, a BTNL8-binding fragment of an antibody is a fragment which retains at least a part of the specific binding activity for BTNL8. In particular, BTNL8-binding fragments of the invention may comprise Fv, Fab, F(ab') and F(ab')2 fragments. Further included by the term "BTNL8-binding fragment" are single chain Fv antibody fragments (which are also designated "scFv antibodies herein) and disulfide-linked Fv fragments (dsFv). Methods for the recombinant production of antibody fragments have been described in the prior art and comprise, for example, techniques such as phage display or ribosome display. The recombinant antibody fragments produced by these methods may be purified and tested for binding affinity and specificity for BTNL8.

In a fifth aspect, the invention relates to a kit for performing the detection method discussed elsewhere herein. The kit comprises the following components:
(a) means for the detection of at least one marker which is known to be associated with Tregs, wherein said marker preferably is CD25, CD127, CD121a, CD121b, GARP, LAP or FoxP3; and
(b) means for the detection of BTNL8.

As discussed above, marker detection can be performed on a transcriptional or translational level. If detection is performed on a transcriptional level, the kit will include at least one polynucleotide primer or probe which is directed to the BTNL8 gene. Preferably, the kit will include more than one polynucleotide primer or probe which is directed to the BTNL8 gene. The primers will allow the detection of expression of the BTNL8 gene. The detection can be performed by PCR or RT-PCR as discussed elsewhere herein. If detection is performed on a translational level, the kit will include at least one antibody or antibody fragment directed to BTNL8, in preferably more than one of such antibody or anybody fragment. The kit may further comprise buffers and reagents that are suitable for the detection method to be performed.

### BRIEF DESCRIPTION OF THE DRAWINGS

Figure 1 shows the results of a qPCR for *BTNL8* mRNA in T cells cocultured with M regs or IFN-y Mcp, CD4+ T cells cultured alone and freshly-isolated CD4+ T cells (n=9; mean ± SEM).
Figure 2 shows the results of an analysis of BTNL8 mRNA expression in activated and unactivated Treg or conventional T cells.
Figure 3 shows a western blot for BTNL8 using a goat anti-BTNL8 pAb from T cells cocultured with allogeneic M regs or IFN-y Mcp and T cells cultured alone.
Figure 4 shows 8 that BTNL8 expression was detected in Mreg-induced Tregs by flow cytometry after staining with an anti-BTNL8 antibody.

### EXAMPLES

The following examples are provided for the illustration of the present invention.

### Example 1: Identification of novel markers associated with Mreg-induced Tregs

In order to better characterize the phenotype of Mreg-induced Tregs, whole genome gene expression profiling studies by microarray were performed. Mregs and IFN-y-Mcp were generated from 5 donors. Unfractionated CD3⁺ T cells were isolated from 5 unrelated, HLA-A2 discrepant donors. A sample of freshly isolated CD3+ T cells was flow-sorted into CD4+ and CD8+ fractions for RNA isolation. Unfractionated T cells were also added into direct co-cultures with Mregs or IFN-y-Mcp at a 1:1 ratio, or were cultured alone for 5 days without stimulation. After 5 days of culture, CD4+ and CD8+ T cells were FACS-sorted and total RNA was extracted. These RNA samples were used to produce to a microarray dataset comprising triplicate samples of eight cell types produced by single-color hybridizations. The heat map showed a hierarchical clustering of reporter sets returned by one-way ANOVA that were significantly (p<0.01) and highly differentially (>20-fold) regulated in any two of the comparator T cell populations. Discriminatory gene analyses (DGA) were performed to identify genes uniquely up-regulated in Mreg-cocultured CD4+ T cells compared to control conditions. BTNL8 was found to be one of the most highly upregulated reporters in allogeneic M reg-cocultured CD4⁺ T cells compared to IFN-y Mcp-cocultured CD4+ T cells.

### Example 2: qPCR for BTNL8 mRNA in T cells cocultured with M regs

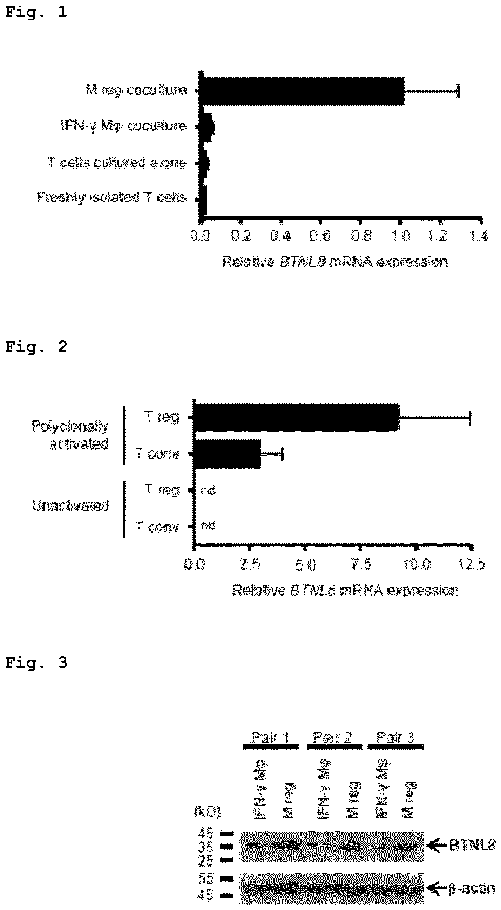

A qPCR for BTNL8 mRNA in T cells was conducted using primers that amplify both the BTN-like and B7-like variants of BTNL8. Specifically, the ready-optimized primer pair BTNL8_2_SG from Qiagen was used (cat.# QT01670991). The T cells had been cocultured with M regs or IFN-y Mcp, CD4+ T cells cultured alone and freshly-isolated CD4+ T cells (n=9; mean ± SEM). SuperScript-III (Invitrogen) was used for reverse-transcription. qPCR was performed with a LightCycler™ real-time PCR system using the FastStart DNA Master SYBR Green I kit (Roche-Diagnostics, Penzberg). Specificity was confirmed by amplicon sequencing (MWG-Biotech, Ebersberg). The results are depicted in Figure 1. Using isoform-specific primer sets and sequencing of amplicons, it was determined that M reg-cocultured T cells express only the B7-like, 37-kD variant of BTNL8. Further, it can be seen there that only T cells which had been cocultured with Mregs express significant amounts of BTNL8.

The expression of *BTNL8* mRNA was then quantified in CD4⁺ CD25⁺ CD12^{int} Tregs and conventional CD4⁺ T cells isolated from human peripheral blood by flow-sorting. For this purpose, human peripheral blood T cells were flow-sorted for CD3+CD4+CD25⁺CD127^{int} Tregs and CD3+CD4+CD25⁺CD127^{int} non-Tregs. The results are depicted in Figure 2. BTNL8 mRNA expression was not detected in unactivated Treg or conventional T cells, but could be induced upon polyclonal activation with aCD3/aCD28 beads for 5 days. The expression of BTNL8 mRNA in activated conventional T cells was rather low compared that in activated Tregs.

### Example 3: Western Blot analysis

A western blot for BTNL8 was performed using a goat a-BTNL8 pAb from T cells cocultured with allogeneic M regs or IFN-y Mcp and T cells cultured alone. Protein G-Plus sepharose (Sigma-Aldrich) was used to immunoprecipitate BTNL8. SDS-PAGE and immunoblotting were performed according to conventional methods.

The results are depicted in Figure 3. A distinct band corresponding to BTNL8 was detected at 37 kD. Examples from 3 donor pairs are shown. Western blotting revealed stronger expression of the 37-kD isoform of BTNL8 in Mreg-cocultured T cells compared to IFN-y-Mcp-cocultured T cells or T cells cultured alone.

### Example 4: Detection of Tregs by flow cytometry

BTNL8 expression was detected in Mreg-cocultured T cells by flow cytometry after staining with a rabbit antibody (LSBio #C453018) that was raised against aa161-210 of BTNL8. The histogram is gated on live CD3+ CD4+ CD8' CD25+ FoxP3+ single events.

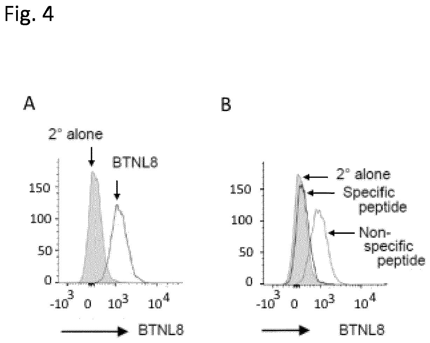

The results are shown in Figure 4. BTNL8 expression was detected in Mreg-induced Tregs by flow cytometry after staining with an anti-BTNL8 antibody. Pre-incubation of rabbit α-BTNL8 pAb with specific peptide (LSBio #E18020) blocked detection of BTNL8, whereas a non-specific peptide corresponding to the N-terminus of BTNL8 (LSBio #E18019) had no effect; therefore, detection of BTNL8 by flow cytometry is specific.

### LITERATURE

[1] Hutchinson & Geissler (2015), Kidney Int.
[2] Ferrer et al. (2014), Immunol. Rev. 258, 102-116.
[3] Trzonkowski et al. (2015), Sci. Transl. Med. 7, 304ps18.
[4] Thomson et al (2016), Front Immunol. 7, 15.
[5] Geissler & Hutchinson (2013), Curr. Opin. Organ Transplant. 18, 408-415.
[6] Riquelme et al. (2012), Transplantation Research 1(1):17
[7] Hutchinson at al. (2011), J. Immunol. 187, 2072-2078
[8] Riquelme et al. (2013), Mol Ther. 2013, 21(2): 409-22
[9] Broichhausen et al. (2012), Curr. Opin. Organ Transplant. 17, 332-342.
[10] Hutchinson et al. (2011), Methods Mol. Biol. 677, 181-192.
[11] Hutchinson et al. (2010), Transplantation 90, 184.
[12] Hutchinson at al. (2011), J. Immunol. 187, 2072-2078.
[13] Riquelme et al. (2013), Mol. Ther. 21, 409-422.
[14] Liston et al. (2013), Adv Immunol., 119:85-106
[15] Battaglia & Roncarolo (2009), Eur J Immunol., 39(12): 3296-300.
[16] Abeler-Dorner et al. (2012), Trends Immunol. 33, 34-41.
[17] Chapoval et al. (2013). Mol. Immunol. 56, 819-828.
[18] Leonard et al. (1985), Science. 228 (4707): 1547-9.
[19] Noguchi et al (1993), Science. 262 (5141): 1877-80.
[20] Kroemer & Richards (1996), Protein Eng. 9 (12): 1135-42.
[21] Ollendorff et al. (1992), Mamm Genome 2:195-200.
[22] Ollendorff et al. (1994), Cell Growth Differ 5:213-219.
[23] Ochi et al. (2006), Nat. Med. 12, 627-635.
[24] Chen et al. (2009), Eur. J. Immunol. 39, 3423-3435.
[25] Liu et al. (2015), PLoS One, 10(6): e0131086.
[26] Garlanda et al. (2013), Immunity 39: 1003-1018.

## Claims

1. Method for detecting regulatory T cells (Tregs) in a subject, said method comprising detecting in a sample that has been obtained from the subject:
(a) at least one marker which is known to be associated with Tregs;
(b) BTNL8.

2. Method of claim 1, wherein said marker which is known to be associated with Tregs is selected from the group of CD25, CD127, CD121a, CD121b, GARP, LAP and FoxP3.

3. Method of any of claims 1-2, wherein said method further comprises the detection of one or more of the following markers: CD3, CD4, TIGIT, and HELIOS.

4. Method of any of claims 1-3, wherein said method comprises the detection of the following markers:
(a) CD4, GARP, and BTNL8,
(b) CD4, LAP, and BTNL8,
(c) CD4, GARP, LAP, and BTNL8,
(d) CD4, CD25, GARP, and BTNL8,
(e) CD4, CD25, LAP, and BTNL8,
(f) CD4, CD25, GARP, LAP, and BTNL8,
(g) CD4, CD127, GARP, and BTNL8,
(h) CD4, CD127, LAP, and BTNL8,
(i) CD4, CD127, GARP, LAP, and BTNL8,
(j) CD4, CD121a, GARP, and BTNL8,
(k) CD4, CD121a, LAP, and BTNL8,
(l) CD4, CD121a, GARP, LAP, and BTNL8,
(m) CD4, CD121b, GARP, and BTNL8,
(n) CD4, CD121b, LAP, and BTNL8,
(o) CD4, CD121b, GARP, LAP, and BTNL8,

5. Method of any of claims 1-4, wherein said method further comprises a step in which the Tregs are quantified.

6. Method of any of claims 1-5, wherein said sample is derived from a subject to whom a cell-based product was administered before detecting said activated Tregs, wherein said cell-based product preferably comprised regulatory macrophages (Mregs).

7. Method of any of claims 1-6, wherein detection in steps (a) and (b) uses one or more of the following techniques: flow cytometry, PCR, RT-PCR, real-time PCR and ELISA.

8. Method of any of claims 1-7, wherein said sample is a blood sample or a blood-derived sample.

9. Method of any of claims 1-7, wherein non-Tregs are used as a negative control.

10. Method for monitoring or evaluating the treatment of an immunological disease or condition in a subject, comprising
(a) providing a sample that has been obtained from said subject after treatment of said immunological disease or condition has been initiated;
(b) carrying out the method of claims 1-9; and
(c) quantifying the BTNL8-expressing Tregs;
wherein an increase in the number of BTNL8-expressing Tregs after initiation of treatment indicates that the treatment is effective.

11. Method for isolating regulatory T cells (Tregs), said method comprising:
(a) providing a sample that contains or is supposed to contain Tregs;
(b) separating Tregs from other cells that are contained in the sample via their expression of BTNL8 and at least one marker which is known to be associated with Tregs, wherein said marker preferably is selected from the group of CD25, CD127, CD121a, CD121b, GARP, LAP and FoxP3.

12. Use of a compound having specific affinity for the BTNL8 protein for detecting, enriching, isolating and/or quantifying Tregs.

13. Use of claim 12, wherein said compound is an antibody or antibody fragment directed to BTNL8.

14. Kit, comprising
(a) means for the detection of at least one marker which is known to be associated with Tregs, wherein said marker preferably is CD25, CD127, CD121a, CD121b, GARP, LAP or FoxP3; and
(b) means for the detection of BTNL8.

15. Kit according to claim 14, wherein said means comprise (i) an antibody or antibody fragment directed to BTNL8, and/or (ii) a polynucleotide primer or probe directed to the BTNL8 gene.

16. Method for detecting regulatory T cells (Tregs) in a pool of T cells, said method comprising detecting in a sample that has been obtained from the subject:
(a) at least one marker which is known to be associated with Tregs;
(b) BTNL8.
